(19) Europäisches Patentamt
European Patent Office
Office européen des brevets

(11) Publication number: 0 274 898
A2

# (12) EUROPEAN PATENT APPLICATION

(21) Application number: 87311363.3

(22) Date of filing: 23.12.87

(51) Int. Cl.4: A61L 27/00

(30) Priority: 27.12.86 DE 3644588

(43) Date of publication of application:
20.07.88 Bulletin 88/29

(84) Designated Contracting States:
BE ES GB IT LU NL SE

(71) Applicant: ETHICON INC.
U.S. Route 22
Somerville New Jersey 08876(US)

(72) Inventor: Hinsch, Bernhard, Dr.
Travestrasse 1
D-2000 Norderstedt(DE)
Inventor: Walther, Christoph, Dr.
AM Brahmberg 19
D-2358 Kattendorf(DE)

(74) Representative: Jones, Alan John et al
CARPMAELS & RANSFORD 43 Bloomsbury Square
London, WC1A 2RA(GB)

(54) **Implant.**

(57) The invention relates to an implant from an open-cell, foam-like plastic material based on resorbable polyesters, such as poly-p-dioxanone, other polyhydroxycarboxylic acids, polylactides or polyglycolides, as well as their copolymers, in which one or more textile reinforcing elements made from resorbable plastic are embedded in an open-cell plastic matrix with a pore size of 10 to 200 µm.

EP 0 274 898 A2

# IMPLANT

The invention relates to porous implants of open-cell, foam-like plastic materials based on resorbable polyesters such as poly-p-dioxanones, polylactides or polyglycolides and their copolymers, as well as to a process for producing the same by the freeze drying of a solution of polymers at low temperatures and under a high vacuum.

Of late, body-resorbable implants have been used in medicine, which offer a temporary support for damaged, injured or missing body parts and which are replaced by the body's own structures to the extent in which they are resorbed. Implants made from compact materials are unsuitable due to the excessive rigidity of the materials and also and in particular because the implants cannot be penetrated by body fluids, cells and blood vessels, so that no new tissue can form.

The textile structure according to US patent 3 739 773 produced from fibres or monofilaments of resorbable plastic, is, as a flat structure, too permeable for body fluids and therefore is unsuitable for many purposes, e.g. for replacing the dura.

In addition, porous, resorbable prostheses are known from DE-OS 32 18 150, DE-OS 33 34 595 and US patent 4 292 972. Apart from pourable ceramic materials, such as e.g. hydroxyl apatite, they relate to biopolymers such as hydrocolloids, gelatins or collagen, as well as synthetic resorbable polymers. However, up to now no product in the latter category has achieved practical significance. Products formed from peptide-containing polymers are unsuitable, because they cause strong foreign body reactions up to repulsion and excapsulation.

As a result of their good physiological compatibility synthetic resorbable polymers, e.g. of aliphatic hydroxycarboxylic acids have been proposed for porous implants, e.g. according to US patent 4 181 983, according to which lactides are polymerized with a catalyst such as tetraphenyl tin, whilst excluding oxygen, at elevated temperatures of 150 to 200°C and in vacuo. Following reprecipitation from dioxan/water in benzene, the polymer obtained is dissolved in a solvent mixture of 30% methanol and 70% benzene and mixed with a surfactant, such as sodium oleate. The polylactide concentration in the final benzene solution is preferably 3.5% by weight and the quantity of the sodium oleate used as the wetting agent is in the range 0.25 to 2.5% by weight. The benzene solution is then frozen in dry ice and freeze dried under high vacuum for 18 hours. However, these products suffer from the disadvantage that through the high molecular weight ($M_w$) of the polylactides of over 100 000 the resorption process takes too long and the pore sizes of under 20 µm obtained are unsuitable, because no body fluids penetrate the pores, no cells migrate in and no blood vessels can grow in, so that no new tissue can form. The necessary addition of detergents can only facilitate the penetration of liquids, but cannot aid the following physiological healing processes. Moreover, surface-active compounds, such as synthetic, anionic, cationic or amphoteric surfactants or soap, which improve the absorbtion of tissue fluids in the foam materials, are not physiologically unobjectionable.

Other conventional processes for producing porous, open-cell, foam-like materials with chemically acting blowing agents are unsuitable, because blowing agent residues are left behind in the foam or reactive products are formed, which can at least partly decompose sensitive polymers, such as resorbable polyesters.

High pressure gassing processes, in which the plastic in solid or pasty form is gassed in a heated mould with a blowing gas mixture and then compressed, prefoamed by pressure reduction following the end of the gelling process and then the foam block obtained is again heated and inflated in a stabilizing furnace are unsuitable, because to obtain a uniform pore size it is necessary to add surface-active compounds and/or nucleating agents, which remain in the foam and are unusable for medical implants.

The problem of the present invention is to propose porous implants and a process for the production thereof, whereby the pores have a suitable size for the growth of blood vessels and cells, namely an average diameter of 10 to 200 and preferably 20 to 150µm, because excessively small pores with a diameter of under 10 µm cannot grow through and large pores lead to a deterioration in the mechanical stability of the porous implants.

According to the invention, it must be possible to produce such open-cell polymers without the use of the adjuvants conventionally employed for foam production, such as surface-active substances, stabilizers, lubricants, physical or chemical blowing agents.

It is finally a problem of the invention to propose implants which, despite the adequately open-cell structure to permit the growing in of cells and blood vessels, have an adequate strength and in particular tensile strength.

Thus, for solving the problem of the invention, an implant of the aforementioned type is proposed, which is constructed in accordance with the characterizing part of the main claim, preferred embodiments

being given in the subclaims. In addition, for solving the inventive problem, processes for producing the implants are proposed.

The invention is based on the surprising finding that the tensile strength of such implants is dependent on the polymer content of the solution to be freeze dried and is only dependent to a reduced extent on the production conditions adopted during freeze drying, such as the freezing rate. However, a part is also played by the choice of solvent and this also influences the pore size distribution, the pore size and the pore shape.

It has surprisingly been found that on incorporating textile reinforcing elements of resorbable plastic such as fibres, yarns, braids, knitted fabrics and the like, it is possible to significantly improve the mechanical strength, without modifying the porosity characteristics, flexibility and elasticity of the foam materials. As a function of the choice of reinforcing elements, the mechanical strength can be increased in one or more directions in space, in that e.g. parallel fibres or threads increase the stability in only one direction and net-like flat structures improve the same in all directions of the corresponding surfaces.

The invention is further illustrated hereinafter by means of examples.

I. Production of the Plastic Matrix

The production of the starting polymers is described hereinafter:

Example A: Polylactides

The starting polymer is produced in that purified lactide crystals are polymerized with 0.002% by weight of tetraphenyl tin, whilst excluding oxygen, for 4 hours in a temperature range of 150 to 200°C, the polymer obtained is precipitated from dioxan by adding water and then dried under a high vacuum. These polylactides have the following general formula:

$$H\text{-}(\text{-}O\text{-}\underset{CH_3}{\underset{|}{CH}}\text{-}\overset{O}{\overset{/\!/}{C}}\text{-}\ )_n\text{-}OH$$

in which n has a value of 300 to 1200 corresponding to a molecular weight of 20 000 to 85 000.

Poly-L-lactide, poly-DL-lactide, as well as copolymers of these two substances and copolymers with glycolides can in particular be used for the inventive products and processes. Apart from glycolides, the polylactides can also contain other monomers, such as are referred to e.g. in US patent 4 181 983, column 3, lines 26 to 36.

Example B: Polyglycolides

The inventively used polyglycolides are obtained by the polymerization of glycolic acid and have the following general formula:

$$H\text{-}(\text{-}O\text{-}CH_2\text{-}\overset{O}{\overset{/\!/}{C}}\text{-}\ )_n\text{-}OH$$

in which n is such that the molecular weight is in a range 10 000 to 500 000. These polyglycolides are prepared in that glycolide is polymerized with tin (II)-octanoate and glycolic acid, whilst excluding oxygen and in vacuo, for approximately 4 to 5 hours at 185 to 230°C and after cooling the polyglycolide is isolated as a white, opaque, viscous material. The inherent viscosity, measured in 1,1,1,3,3,3-hexafluoro-2-propanol is in the range 0.5 to 4.

Example C: Copolymers of Glycolide and Lactide

9 parts by weight of glycolide and 1 part by weight of L-lactide are polymerized with glycolic acid and tin (III)-caprylate, as described in DE-OS 21 62 900, whilst excluding oxygen and moisture for approximately 4 hours at 200°C. The inherent viscosity of the polymer is 0.5 to 2.5 when measured in 1,1,1,3,3,3-hexafluoro-2-propanol.

Block copolymers of glycolide and lactide can also be used for the inventive products and processes, as described in DE-OS 28 49 785.

Example D: Polydioxanone

The inventively used polydioxanone has the following general formula

$$H-\left[O-CH_2-CH_2-O-CH_2-\overset{O}{\overset{\|}{C}}\right]_n-OH$$

Polydioxanone is polymerized by the ring-opening polymerization of 2-oxo-1,4-dioxan in the presence of Sn catalists, such as e.g. tin (II)-caprylate at 120 to 150°C, whilst excluding oxygen and moisture, within 1 to 4 hours. The inherent viscosity (measured in 1,1,1,3,3,3-hexafluoro-2-propanol) of the polymer is in the range 0.5 to 3 and preferably 1.5 to 2.2.

II. Preparation of the Plastic Solution or Dispersion

Decisive for the preparation of the plastic matrix are the solvent chosen and the concentration of the resorbable plastic in the solvent to be freeze dried and optionally, in the case of a modified process, the addition of crystalline additives. Other solvents can be used, particularly in the latter case.

A. Solvent influence

According to the invention use is made of the following solvents:

a) 1,4-dioxan

b) 1,4-dioxan with an acetate of a $C_2$ to $C_5$ alcohol, namely ethyl acetate in the ratio 9:1,

c) hexafluoroisopropanol and as the comparison solvent

d) benzene with and without surfactant.

In each case 5 g of a poly-L-lactide according to example A are introduced into 100 g of the particular solvent and frozen from 20°C to -60°C in between 30 and 300 seconds and then freeze dried in vacuo.

The following table I shows the influence of the solvent selection on the pore structure of the polymer matrix.

TABLE I

Influence of the Solvent Selection on the Pore Structure

| | Solvent | Pore Characterization |
|---|---|---|
| A-a | dioxan | open, round pores : diameter 20-50 µm |
| A-b | dioxan/ethylacetate 9:1 | open, round pores : diameter 20-60 µm |
| A-c | hexafluoroisopropanol | open, round pores, fibrous : diameter 10-50 µm |
| A-$d_1$ | benzene | long channels with a diameter under 10 µm, a few oval pores of 20 - 100 µm |
| A-$d_2$ | benzene + 2.5% surfactant | elongated pores (fibrous product); diameter 5-25 µm |

The values of table I clearly show that only the inventively used solvents lead to a suitable polymer matrix. Similar results were obtained with the other polymerizable polymers according to example B (polyglycolides), example C (copolymers) and example D (polydioxan), as is shown by table II.

B. Influence of the Concentration

Different polymers in different concentrations were treated in a solvent mixture of dioxan and ethyl acetate in a ratio of 9:1 as in A-b) and freeze dried. The following table III shows that excellent pore sizes and distributions are obtained on respecting a concentration in the middle of the inventive concentration range.

TABLE II

Influence of the Solvent Selection on the Pore Structure of Copolymers of L-Lactide and Glycolide

| Glycolide | Lactide | Solvent | Pore Characterization |
|---|---|---|---|
| (Weight Ratio in the Copolymer) | | | |
| 90 | 10 | HFIP | Fibrous, round, open pores, diameter approximately 20 - 100µm |
| 30 | 70 | dioxan/ethylacetate 9:1 | Round, open pores with a diameter of 20 - 50 µm |

TABLE III

Influence of the Polymer Concentration on the Pore Size

| Polymer | Concentration (g/100 ml solvent) | Pore Characterization | | |
|---|---|---|---|---|
| Poly-L-lactide | 5 | open, round pores; | diameter | 20- 50µm |
| | 7.5 | open, round pores; | diameter | 20-100µm |
| | 12 | open, round pores; | 80% of the pores<br>20% of the pores ≤ | 20-100µm<br>20µm |
| | 16 | open, round pores; | 10% of the pores<br>90% of the pores ≤ | 20- 30µm<br>20µm |
| Copolymer of glycolide and lactide 3:7 | 12 | open, round to elongated pores; | 80% of the pores<br>20% of the pores < | 20-100µm<br>20 or > 100µm |
| Copolymer of glycolide and lactide 3:7 | 7.5 | open, round to elongated pores; | diameter | 20- 80µm |
| Poly-D,L-lactide | 7.5 | open, round pores; | 88% of the pores<br>10% of the pores ≤<br>2% of the pores | 20-100µm<br>20µm<br>100-150µm |

C. Adjusting the Pore Size Distribution by Crystalline Additives

4 parts of a copolymer of L-lactide and glycolide (9:1) and 1 part of citric acid are pulverized, screened and classified, the mixture being dissolved and/or suspended with 40 parts of 1,1,1,3,3,3-hexafluoro-2-propanol. As described in A, this mixture is frozen and freeze dried.

The citric acid is extracted with tetrahydrofuran from the resulting crude foam.

The foam contains circular, open pores, whose pores sizes are largely dependent on the particle size distribution of the citric acid used. An example is given in table IV.

TABLE IV

Influence of Crystalline Additives on the Pore Structure

| Particle Size | Citric Acid | Pore Size in Foam | |
|---|---|---|---|
| 80% | 20µm to 40µm | 75% | 70µm to 40µm |
| 80% | ≤ 20µm | 90% | ≤ 20µm |

III. Production of the Implants

Example 1

Different solutions of 2.0 to 30.0 g of poly-L-lactide are in each case dissolved in 100 ml of 1,4-dioxan/ethylacetate (90:10). A reinforcing element of the copolymer of L-lactide and glycolide in the weight ratio of 9:1 is in each case introduced into these solutions. The net in a dish is then frozen for 30 to 90 seconds to -60°C and then freeze dried.

Table V shows the value of the tensile strength of the polymer matrix without reinforcing inserts, determined with a tensiometer at 50 mm/min and a 2x10x50 mm test piece, the tensile strength through the surface of fracture gave the indicated tensile strength.

The tests revealed a rise in the tensile strength of the polymer matrix with increasing polymer concentration (no. 1-4) and under lower freezing conditions (test 5), as well as the comparatively inferior values in the case of a conventional solvent (tests 2 and 6 compared with test 7).

## TABLE V

### Tensile Strength of Porous Implants Without Reinforcing Elements

| Test No. | Polymer Concentration (g/100ml of solvent) | Tensile Strength $N/mm^2$ | Freezing Conditions | Solvent |
|---|---|---|---|---|
| 1 | 4 | 0.10 | A | 1,4-dioxan/ethyl acetate 9:1 |
| 2 | 6 | 0.35 | A | |
| 3 | 10 | 0.90 | A | |
| 4 | 15 | 1.95 | A | |
| 5 | 4 | 0.28 | B | |
| 6 | 6 | 0.53 | A | 1,4-dioxan/ isoamyl acetate 19:1 |
| 7 | 6 | 0.30 | A | benzene |

A 20°C to -60°C in 30-300 seconds

B 20°C to -130°C in 30-300 seconds

Table VI shows the surprising increase in the tensile strength when using textile reinforcing elements in a polymer matrix according to example 1.

## TABLE VI

### Tensile Strength of Reinforced Porous Implants

| Polymer Concentration (g/100ml of solvent) | Tensile Strength $N/mm^2$ | Reinforcing Element |
|---|---|---|
| 4 | 0.10 | without |
| 4 | 1.35 | 2dpf yarn, length 2-5 cm |
| 4 | 8.0 | knitted net |
| 10 | 1.30 | 2dpf yarn, 1-5 mm |
| 10 | 10.2 | knitted net |

Table VII shows the increase in the tensile strength of porous tubular implants with woven reinforcing elements of different weaving structures.

## TABLE VII

### Breaking Force of Porous Implants with Woven Reinforcing Elements

| Breaking Force | Reinforcing Element |
|---|---|
| 1.4N | none |
| 40 | thick-walled hose, 2x56 denier threads |
| 70 | impervious hose, 4x56 denier threads |
| 150 | very thick-walled, impervious hose, 8x56 denier threads |

| | |
|---|---|
| Test pieces: | external diameter 4 mm<br>internal diameter 2.7 mm |
| Polymer concentration: | 5g/100ml of solvent |
| Reinforcing element: | copolymer of L-lactide and glycolide 1:9 |

**Claims**

1. Implant of an open-cell, foam-like plastic material based on resorbable polyesters such as poly-p-dioxanone, other polyhydroxy carboxylic acids, polylactides or polyglycolides, as well as their copolymers, characterized in that one or more reinforcing elements of a textile nature formed from resorbable plastic are embedded in an open-cell plastic matrix with a pore size of 10 to 200 µm.

2. Implant according to claim 1, characterized in that compared with the resorbable plastic of the matrix, the resorbable plastics of the textile reinforcing elements have the same or a slower resorbability.

3. Implant according to claim 1, characterized in that the textile reinforcing elements are knitted, woven, twisted, braided or as felts in the form of fibres, threads, hoses, strips or fleeces.

4. Implant according to claims 1 to 4, characterized in that the open-cell plastic matrix has a pore size of 20 to 150 µm.

5. Implant according to claims 1 to 3, characterized in that the density of the plastic matrix is 0.05 to 0.60 g/cm$^3$.

6. Process for the production of an implant according to claims 1 to 5, characterized in that poly-p-dioxanone, polylactides or polyglycolides are dissolved in a solvent in a concentration of 5 to 30 parts by weight of polymer and the textile reinforcing element is frozen in a mould together with the plastic solution and then the solvent is removed by freeze drying.

7. Process according to claim 6, characterized in that the solvent used is hexafluoroisopropanol, 1,4-dioxan or a mixture of 1,4-dioxan and an acetate of a $C_2$ to $C_5$ alcohol in a volume ratio of 99:1 to 50:50.

8. Process according to claims 6 and 7, characterized in that use is made of poly-p-dioxanone with an inherent viscosity of 0.5 to 3.0 or polylactides with an inherent viscosity of 0.5 to 2.2 or polyglycolides with an inherent viscosity of 0.5 to 4.0.

9. Modification of the process according to claims 6 to 8, characterized in that the textile reinforcing element is impregnated with the plastic solution and then frozen and freeze dried.

10. Modification of the process according to claims 6 to 8, characterized in that crystalline organic compounds, salts of organic acids or inorganic salts are added to the solvents and after freeze drying are extracted from the implant with a suitable inert solvent.